# EUROPEAN PATENT APPLICATION

(11) **EP 2 420 253 A1**
(43) Date of publication of application: **22.02.2012**
(21) Application number: 10382234.2
(22) Date of filing: 20.08.2010
(51) Int. Cl.: A61K 47/48, C07K 14/78

(54) **Engineering multifunctional and multivalent molecules with collagen XV trimerization domain**

(71) Applicant: Leadartis, S.L., 28008 Madrid (ES)
(72) Inventor: Álvarez Vallina, Luis, 28008 Madrid (ES); Cuesta Martínez, Ángel, 28008 Madrid (ES); Sainz Pastor, Noelia, 28008 Madrid (ES); Sanz Alcober, Laura, 28008 Madrid (ES)
(74) Representative: ABG Patentes, S.L.

(57) **Abstract**

The invention relates generally to a new trimerization domain useful for developing multifunctional and multivalent complexes. Particularly, the invention relates to the design of trimeric polypeptide complexes using polypeptide structural elements derived from the collagen XV protein, and their use in diagnostic and therapeutic systems *in vivo* and *in vitro.* The invention also relates to nucleic acids and vectors useful for producing said trimeric complexes.

SEQUENCE LISTING
<110>  LeadArtis S.L.

<120>  ENGINEERING MULTIFUNCTIONAL AND MULTIVALENT MOLECULES WITH
       COLLAGEN XV TRIMERIZATION DOMAIN

<130>  P6066EP00

<160>  13

<170>  PatentIn version 3.5

<210>  1
<211>  64
<212>  PRT
<213>  Artificial Sequence

<220>
<223>   collagen XV trimerizing structural element  (XVTSE)

<400>  1

Val Thr Ala Phe Ser Asn Met Asp Asp Met Leu Gln Lys Ala His Leu
1               5                   10                  15

Val Ile Glu Gly Thr Phe Ile Tyr Leu Arg Asp Ser Thr Glu Phe Phe
            20                  25                  30

Ile Arg Val Arg Asp Gly Trp Lys Lys Leu Gln Leu Gly Glu Leu Ile
        35                  40                  45

Pro Ile Pro Ala Asp Ser Pro Pro Pro Pro Ala Leu Ser Ser Asn Pro
    50                  55                  60

<210>  2
<211>  192
<212>  DNA
<213>  Artificial Sequence

<220>
<223>  XVTSE DNA sequence

<400>  2
gtgaccgcct tcagcaacat ggacgacatg ctgcagaagg cccacctggt catcgagggc       60

accttcatct acctgcggga cagcaccgag ttcttcatca gagtgcggga cggctggaag      120

aagctgcagc tgggcgagct gatccccatc cccgccgata gccctccacc tcctgccctg      180

agcagcaacc ct                                                          192

<210>  3
<211>  14
<212>  PRT
<213>  Artificial Sequence

<220>
<223>  polyglycine linker

<400>  3

Ser Gly Gly Thr Ser Gly Ser Thr Ser Gly Thr Gly Ser Thr
1               5                   10


<210>  4
<211>  15
<212>  PRT
<213>  Artificial Sequence

<220>
<223>  polyglycine linker

<400>  4

Ala Gly Ser Ser Thr Gly Ser Ser Thr Gly Pro Gly Ser Thr Thr
1               5                   10                  15


<210>  5
<211>  7
<212>  PRT
<213>  Artificial Sequence

<220>
<223>  polyglycine linker

<400>  5

Gly Gly Ser Gly Gly Ala Pro
1               5


<210>  6
<211>  8
<212>  PRT
<213>  Artificial Sequence

<220>
<223>  polyglycine linker

<400>  6

Gly Gly Gly Val Glu Gly Gly Gly
1               5


<210>  7
<211>  4
<212>  PRT
<213>  Artificial Sequence

<220>
<223>  flexible linker

<400>  7

Gly Ser Pro Gly
1


<210>  8
<211>  13
<212>  PRT
<213>  Artificial Sequence

<220>
<223>  flexible linker

<400>  8

Ala Ala Ala Gly Gly Ser Gly Gly Ser Ser Gly Ser Arg
1               5                   10


<210>  9
<211>  8
<212>  PRT
<213>  Artificial Sequence

<220>
<223>  flexible linker

<400>  9

Gly Ser Gly Ser Gly Ser Gly Ser
1               5


<210>  10
<211>  21
<212>  PRT
<213>  Artificial Sequence

<220>
<223>  flexible linker

<400>  10

Ala Asn Ser Gly Ala Gly Gly Ser Gly Gly Ser Ser Gly Ser Asp Gly
1               5                   10                  15


Ala Ser Gly Ser Arg
            20


<210>  11
<211>  10
<212>  PRT
<213>  Artificial Sequence

<220>
<223>  10 amino acid residues of the upper hinge region of murine IgG3

<400>  11

Pro Lys Pro Ser Thr Pro Pro Gly Ser Ser
1               5                   10


<210>  12
<211>  11
<212>  PRT
<213>  Artificial Sequence

<220>
<223>  spacer

<400>  12

Ala Pro Ala Glu Thr Lys Ala Glu Pro Met Thr
1               5                   10


<210>  13
<211>  5
<212>  PRT
<213>  Artificial Sequence

<220>
<223>  spacer

<400>  13

Ala Ala Ala Leu Glu
1               5

## Description

### FIELD OF THE INVENTION

The invention relates generally to a new trimerization domain useful for developing multifunctional and multivalent complexes. Particularly, the invention relates to the design of trimeric polypeptide complexes using polypeptide structural elements derived from the collagen XV protein, and their use in diagnostic and therapeutic systems *in vivo* and *in vitro.* The invention also relates to nucleic acids and vectors useful for producing said trimeric complexes.

### BACKGROUND OF THE INVENTION

Conversion of monovalent recombinant antibodies, such as scFv (single-chain variable fragments), Fabs (fragment antigen binding) or single-domains, into multivalent formats increases functional affinity, decreases dissociation rates when bound to cell-surface antigens, and enhances biodistribution (for a comprehensive review see Cuesta AM et al. Trends Biotech. 2010, 28:355-62).

The most common strategy to create multivalent antibodies has been the engineering of fusion proteins in which the antibody fragment makes a complex with homodimerization proteins. The most relevant recombinant dimeric antibodies are: the Fab₂ antibody and its single-chain counterpart, sc(Fab')₂ (Fab₂/sc(Fab)₂); the ZIP miniantibody; the scFv-Fc antibody; and the minibody. The miniantibody is a 64 kDa molecule generated by the association of two scFv fragments through dimerization domains. Leucine zippers (Fos or Jun) are employed to mediate dimerization of scFv in a miniantibody molecule. Fos―Fos and Jun―Jun homodimer formation allow efficient production of stable, secreted, monospecific miniantibodies, and Fos―Jun heterodimer formation allows production of bispecific miniantibodies. The scFv-Fc antibody is a small IgG-like recombinant antibody (100―105 kDa) generated by fusion of a scFv with human IgG₁ hinge and Fc regions (C_{H}2, and C_{H}3 domains). The 80 kDa minibody results from the fusion of a scFv to the human IgG₁ C_{H}3 domain. Other minibody-like molecules have been generated using the small immunoprotein technique, or by fusion to bacterial alkaline phosphatase.

Multimerization can be forced by shortening the peptide linker between the V domains of the scFv to self-associate into a dimer (diabody; 55 kDa). Further diabody derivatives are the triabody and the tetrabody, which fold into trimeric and tetrameric fragments by shortening the linker to less than 5 or to 0―2 residues, respectively. A different design for a (scF_{V})₂ is the 'bispecific T cell engager' (BITE). BITEs are bispecific single-chain antibodies consisting of two scFv antibody fragments, joined via a flexible linker, that are directed against a surface antigen on target cells and CD3 on T cells.

Oligomerization domains from non-Ig proteins have been used to make multivalent antibodies (i.e. molecular constructs based on the bacterial barnase―barstar module). The ribonuclease barnase (12 kDa) and its inhibitor barstar (10 kDa) form a very tight complex in which the N- and C-termini are accessible for fusion. This module, which has a dissociation constant of about 10^{―14} M, has been exploited to generate stable, trimeric scFv (about 130 kDa). Similar formats have been generated by fusion of a scFv to a cytokine that naturally exists in trimeric form, the tumor necrosis factor (TNF). TNFα genetically fused to the C-terminus of a scFv antibody forms a homotrimeric structure that retains both TNFα activity and the ability to bind antigen recognized by the scFv. Also, fusion proteins of scFv fragments with the C-terminal multimerization domain of the tumor-suppressor protein p53 or with a streptavidin subdomain results in tetrameric antibodies (scFv-SA)₄.

Another successful approach for the generation of multimeric antibodies has been the use of extracellular matrix-derived oligomerization domains. Trimerization of heterologous fusion proteins containing collagenous domain(s) has been accomplished by employing either a homogeneous or heterologous trimerization domain fused to the collagenous domain to drive the collagen triplex formation. Examples of a trimer-oligomerizing domain include a C-propeptide of procollagens, a coiled-coil neck domain of collectin family proteins, a C-terminal portion of FasL and a bacteriophage T4 fibritin foldon domain [Frank et al., (2001) J Mol Biol 308: 1081-1089; Holler et al., (2003) Mol Cell Biol 23: 1428-1440; Hoppe et al., (1994) FEBS Lett 344: 191-195)]. A trimeric antibody, collabody (125 kDa), is based on the use of triplex-forming collagen-like peptides [(GPP)₁₀].

Fusion of the trimerization region of the C-terminal non-collagenous domain (NC1) of collagen XVIII to the C-terminus of a scFv confers a natural trimeric state to the fused antibody (trimerbody; 110 kDa). WO 2006/048252 discloses fusion proteins comprising the scFv fragment of an antibody and the NC1 domain of collagen XVIII, said domain comprising a trimerization domain and an endostatin (ES) domain bound by a hinge peptide containing proteinase-sensitive sites.

However, the stability of recombinant antibody-based molecules containing the trimerization region from collagen XVIII NC1 domain in serum is not very high; further, the stability of said recombinant antibody-based molecules in the presence of proteases decreases drastically, consequently, the eventual use of said molecules for uses *in vivo* is seriously limited, because the stability of engineered antibody fragments in serum and in protease-rich environment (e.g., tumors, inflammatory sites, etc.) are the critical parameters to determine their potential application *in vivo.*

Therefore, there is a need in the prior art to provide recombinant molecules containing a trimerization domain having a high stability in serum and in the presence of proteases.

The inventors have found that recombinant molecules containing the trimerization region from collagen XV NC1 domain are trimeric in solution, and significantly more stable in serum and in the presence of proteases than recombinant molecules containing the trimerization region from collagen XVIII NC1 domain.

### BRIEF DESCRIPTION OF THE FIGURES

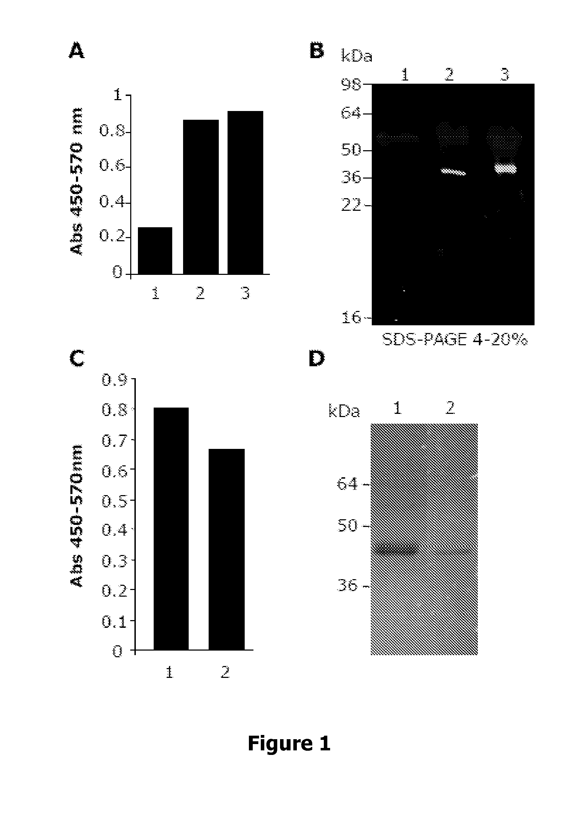

**Figure 1****.** The presence of secreted recombinant antibodies in the supernatant of gene modified 293 cells was demonstrated by ELISA (A) against plastic immobilized laminin (1, L36 scFv; 2, L36-mXVIIINC1ES-; and 3, L36-hXVNC1ES-) and by Western blot analysis (B) (1, untransfected cells; 2, L36-mXVIIINC1ES-; and 3, L36-hXVNC1ES-). Migration distances of molecular mass markers are indicated (kDa). The blot was developed with anti-myc moclonal antibody. (C) Analysis of the purified proteins was demonstrated by ELISA (A) against plastic immobilized laminin (1, L36-mXVIIINC1ES-; and 2, L36-hXVNC1ES-) and by SDS-PAGE analysis (D) (1, L36-mXVIIINC1ES-; and 2, L36-hXVNC1ES-).

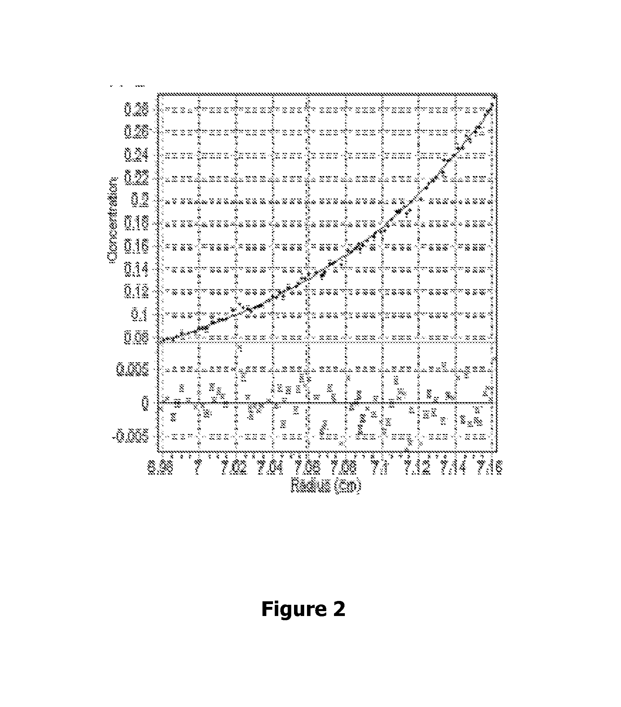

**Figure 2****.** Sedimentation equilibrium gradient of L36- hXVNC1ES- (1 µg/ml in PBS) at 11,000 rpm and 20°C. Closed circles represent the experimental data, and the solid line represent the theoretical gradient of a trimer (117.6 kDa).

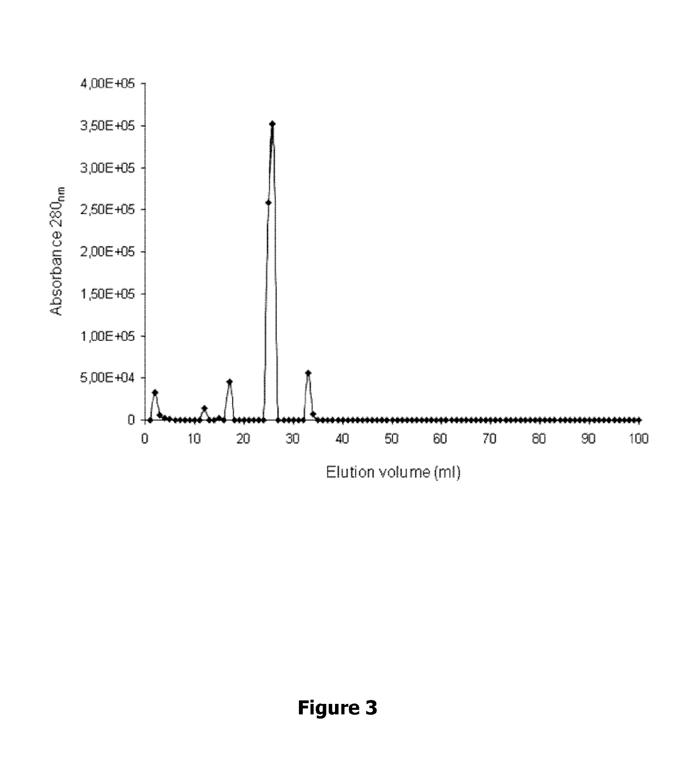

**Figure 3****.** Sedimentation velocity distribution of the L36-hXVNC1ES- (1 µg/ml in PBS) at 42,000 rpm and 20°C.The main peak represents the elution time of the L36-hXVNC1ES- recombinant antibody, showing a molecular weight of 117.605 kDa.

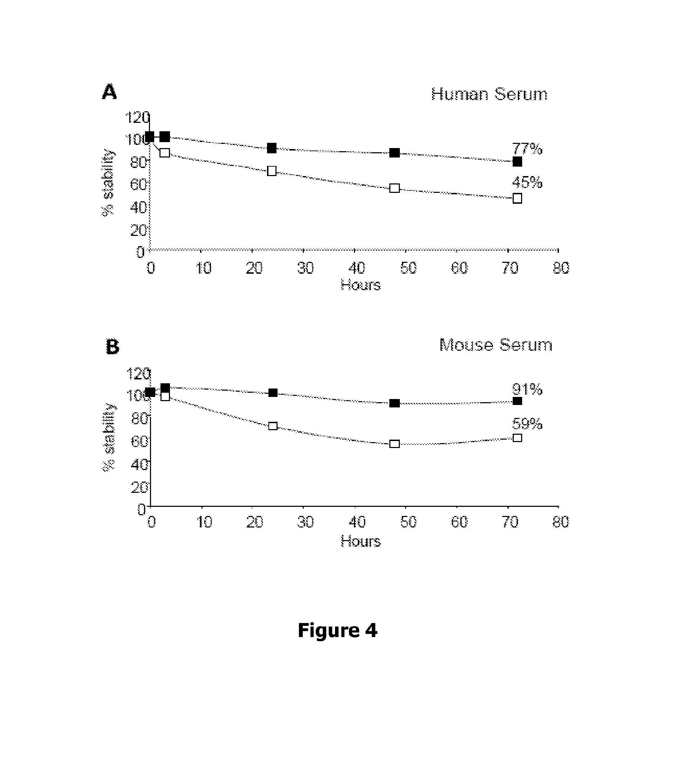

**Figure 4****.** Comparative serum stability of purified L36-mXVIIINC1ES- (open squares) and L36-hXVNC1ES- (filled squares), after incubation at 37°C in human (A) or mouse (B), as explained in material and methods. At the indicated times the reaction mixtures were analyzed by ELISA against plastic immobilized laminin. The percentage of functional active antibody at 72 h time point is shown.

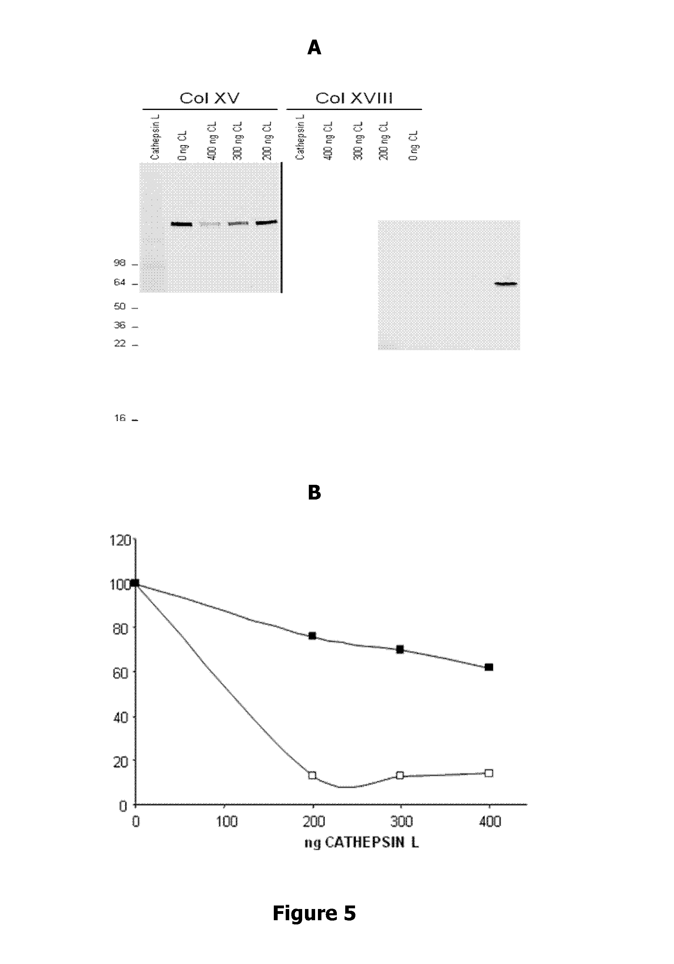

**Figure 5****.** Comparative protease stability of purified L36-mXVIIINC1ES- (open squares) and L36-hXVNC1ES- (filled squares), after incubation at 37°C with different quantities of cathepsin L as explained in material and methods. The reaction mixtures were analyzed by Western blot (A) and ELISA (B) against plastic immobilized laminin. The percentage of functional active antibody after the incubation time point is shown.

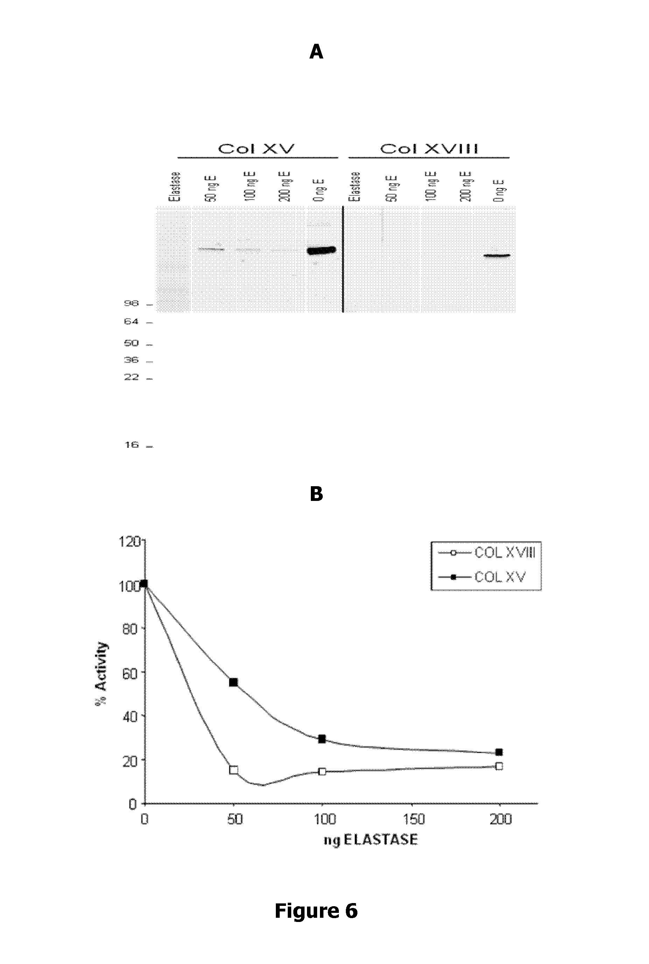

**Figure 6****.** Comparative protease stability of purified L36-mXVIIINC1ES- (open squares) and L36-hXVNC1ES- (filled squares), after incubation at 37°C with different quantities of elastase as explained in material and methods. The reaction mixtures were analyzed by Western blot (A) and ELISA (B) against plastic immobilized laminin. The percentage of functional active antibody after the incubation time point is shown.

### DETAILED DESCRIPTION OF THE INVENTION

The inventors have found that a collagen XV trimerizing structural element (XVTSE) forms, surprisingly, highly stable trimeric molecules. Stability of proteins in serum is a critical parameter to determine its potential application *in vivo;* therefore, said trimeric molecules may find a lot of applications in the field of therapy and diagnosis.

Thus, in an aspect, the invention relates to a trimeric polypeptide complex, hereinafter referred to as the "trimeric complex of the invention", comprising three monomer polypeptides, wherein (i) each of said monomer polypeptides comprises a collagen XV trimerizing structural element (XVTSE) and (ii) at least one of said monomer polypeptides is covalently linked to at least one heterologous moiety.

As used herein, the term "collagen XV trimerizing structural element" or "XVTSE", refers to the portion of collagen XV which is responsible for trimerization between monomers of collagen XV. The term is also intended to embrace functionally equivalent variants of a XVTSE of a naturally occurring collagen XV, variants which have been modified in the amino acid sequence without adversely affecting, to any substantial degree, the trimerization properties relative to those of the native collagen XV molecule. Said modifications include, the conservative (or non-conservative) substitution of one or more amino acids for other amino acids, the insertion and/or the deletion of one or more amino acids, provided that the trimerization properties of the native collagen XV protein is substantially maintained, i.e., the variant maintains the ability (capacity) of forming trimers with other peptides having the same sequence at physiological conditions. The ability of a functionally equivalent variant to form trimers can be determined by conventional methods known by the skilled person in the art. For example, by way of a simple illustration, the ability of a functionally equivalent variant to form a trimer can be determined by using standard chromatographic techniques. Thus, the variant to be assessed is put under suitable trimerization conditions and the complex is subjected to a standard chromatographic assay under non denaturing conditions so that the eventually formed complex (trimer) is not altered. If the variant trimerizes properly, the molecular size of the complex would be three times heavier than the molecular size of a single molecule of the variant. The molecular size of the complex can be revealed by using standard methods such as analytical centrifugation, mass spectrometry, size-exclusion chromatography, sedimentation velocity, etc.

Although said XVTSE can derive virtually from any subject, in a particular embodiment said subject is a mammal, such as a mouse, a rat, a monkey, etc., preferably a human being. Thus, in a particular embodiment, the XVTSE is derived from human collagen XV. Specially preferred is a monomer polypeptide comprising at least one XVTSE derived from human collagen XV.

In a particular embodiment, said XVTSE consists of, or comprises, the N-terminal trimerization region of the collagen XV NC1 domain, such as the region comprising from residue 1133 to residue 1388 of human collagen XV, preferably from residue 1133 to residue 1198. Two sequences for the NC1^{ES-} "trimerization domain" from collagen XV can be found: (i) the first one can be found by annealing the protein sequence from the NC1^{ES-} "trimerization domain" of the collagen XVIII with the collagen XV protein sequence; and (ii) the second one appears disclosed by Boudko [Sergei P. Boudko, "Crystal Structure of Human Collagen XVIII Trimerization Domain, A Novel Collagen Trimerization Fold", JMB, Volume 392, Issue 3, 25 September 2009, Pages 787-802]. The trimeric structure of NC1 is mainly stabilized by a central hydrophobic core, where no solvent molecules are observed.

In another particular embodiment, the XVTSE is a polypeptide having the amino acid sequence shown in SEQ ID NO: 1 [VTAFSNMDDMLQKAHLVIEGTFIYLRDSTEFFIRV RDGWKKLQLGELIPIPADSPPPPALSSNP] or a functionally equivalent variant thereof, i.e., a peptide derived from the peptide whose amino acid sequence is shown in SEQ ID NO: 1 by means of modification, insertion and/or deletion of one or more amino acids, provided that the trimerization property of the native collagen XV protein, namely from its NC1 domain, is substantially maintained, i.e., the functionally equivalent variant maintains the ability of the peptide whose amino acid sequence is shown in SEQ ID NO: 1 of forming trimers with other peptides having the same trimerization domain at physiological conditions. The ability of the functionally equivalent variant to form trimers can be determined by conventional methods as discussed above.

Illustrative, non-limitative, examples of functionally equivalent variants are those showing a degree of identity with respect to the peptide whose amino acid sequence is shown in SEQ ID NO: 1 equal to or greater than at least 30%, at least 40%, at least 50%, at least 60%, at least 70%, at least 80%, at least 90%, at least 95%, at least 96%, at least 97%, at least 98% or at least 99%. The degree of identity between two amino acid sequences can be determined by any conventional method, for example, by means of standard sequence alignment algorithms known in the state of the art, such as, for example BLAST (Altschul S.F. et al. Basic local alignment search tool. J Mol Biol. 1990 Oct 5; 215(3):403-10). In a particular embodiment, the XVTSE is a functionally equivalent variant of the polypeptide having the amino acid sequence shown in SEQ ID NO: 1, said functionally equivalent variant having at least 80%, preferably at least 90%, more preferably at least 95% amino acid sequence identity with the sequence shown in SEQ ID NO: 1.

The person skilled in the art will understand that the amino acid sequences referred to in this description can be chemically modified, for example, by means of chemical modifications which are physiologically relevant, such as, phosphorylations, acetylations, etc.

It has been found that the XVTSE forms surprisingly stable trimeric molecules even more stable than trimeric molecules comprising the N-terminal trimerization region of the collagen XVIII NC1 domain (Example 1). As it is well-known, the stability of proteins in serum and in protease-rich environments is a critical parameter to determine its potential application *in vivo.* As it is shown in Figure 4, the purified L36 trimeric complex containing the collagen XV NC1 trimerization region [L36-hXVNC1ES-] is more stable than the L36 trimeric complex containing the collagen XVIII NC1 trimerization region [L36-mXVIIINC1ES-] (77%-91% vs 45-59%, in human or mouse serum, respectively). Further, said purified L36-hXVNC1ES- is more stable than said L36-mXVIIINC1ES- in the presence of 200 ng of cathepsin L [75,6% vs 13,1%, respectively (Figure 5)] as well as in the presence of 50 ng of elastase [55% vs 14,7%, respectively (Figure 6)].

By the term "heterologous moiety" is herein meant any chemical entity that can be fused or linked covalently to a XVTSE and to which the XVTSE is not natively covalently bound. Hence, the heterologous moiety can be any covalent partner moiety known in the art for providing desired binding, detection, or effector properties. Illustrative, non-limitative, examples of heterologous moieties include:
a) a ligand binding structure, such as a receptor molecule or the ligand binding part of a receptor molecule, such as for example a truncated form of the vascular endotelial factor receptor (VEGFR) 1 or 2; an antibody, an antigen binding antibody fragment, or a molecule having antibody characteristics, such as, for example, a single chain Fv (scFv), a diabody, a domain antibody (V_{H}), a nanobody (V_{H}H), binding molecules derived from the so-called alternative scaffolds, such as DARPins, anticalins, affibodies, adnectins, finomers, etc., or other ligand binding molecules such as avidin or streptavidin, or a lectin, etc.
b) a toxin such as ricin, Pseudomonas exotoxin A, etc.;
c) a detectable label such as a fluorescence labelled molecule, a radioactively labelled molecule, or an enzymatically labelled molecule;
d) an *in situ* activatable substance, such as a molecule which can be induced by a magnetic field or by radiation to be radioactively or chemically active;
e) an enzyme such as a peroxidase, etc.;
f) a radioactive moiety such as a γ-, α-, β-, or β⁺-emitting molecule, e.g. a molecule comprising one or more radioactive isotopes selected from ¹⁴C, ³H, ³²P, ³³P, ²⁵S, ³⁸S, ³⁶Cl, ²²Na, ²⁴Na, ⁴⁰K, ⁴²K, ⁴³K, and any isotopes conventionally utilized for the purposes of facilitating detection of probes or the purposes of providing localized radiation so as to effect cell death;
g) a cytokine such as an interferon, an interleukin (e.g., interleukin-12, etc.), a leukotriene, an angiogenic growth factor such as vascular endotelial factor (VEGF), etc.;
h) an angiogenesis inhibitor such as endostatin, angiostatin, etc.
i) a Peptide Nucleic Acid (PNA);
j) a non-proteinaceous polymer such as a polymeric alkaloid;
k) a polyalcohol;
l) a polysaccharide;
m) a lipid;
n) a polyamine;
o) a photo cross-linking moiety, i.e., a chemical entity which effects cross-linking upon photo-activation;
p) a group facilitating conjugation of the monomer polypeptide to a target; or
q) a tag, i.e., a tag for the purpose of facilitating the isolation and purification of the trimeric complex of the invention, for example an affinity purification tag such as a tag peptide; illustrative, non-limitative examples of said tags include polyhistidine [poly(His)] sequences, peptide sequences capable of being recognized by antibodies that may be used to purify the resultant fusion protein by immunoaffinity chromatography, for example epitopes derived from the hemagglutinin of the fever virus or C-myc epitope, Strep tag, etc.

In a particular embodiment, said heterologous moiety is an antibody, for example a monoclonal antibody (mAb), or a recombinant fragment thereof, e.g., a single chain Fv fragment (scFv), a single domain antibody, etc. In a particular embodiment, said heterologous moiety is the scFv L36 (Example 1) containing the variable heavy chain region (V_{H}) fused with a (Gly₄Ser)₃ linker to the variable light chain (V_{L}), whose sequence has been described by Sanz L et al. [Cancer Immunology and Immunotherapy, 2001 Dec; 50(10)557-65], wherein the C-terminus of said L36 scFv is linked to the N-terminus of said XVTSE, namely to the N-terminus of the human collagen XV NC1 trimerization region [L36-hXVNC1ES-], through a flexible peptide spacer. The scFv- L36 recognizes laminins of different animal species, for example, from mice, rats, humans, etc., since it interacts with a region that is very preserved among different animal species [Sanz L et al. EMBO J 2003, Vol. 22(7):1508-1517].

In another particular embodiment, the heterologous moiety comprises a specific affinity purification tag in order to obtain a substantially pure trimeric complex of the invention, particularly if each one of the three monomer polypeptides comprises one affinity purification tag, said tags being different each other (e.g., affinity purification tags "a", "b" and "c", wherein tag "a" is recognized by binding substance A, tag "b" is recognized by binding substance B, and tag "c" is recognized by binding substance C), and it is subjected to a three-step affinity purification procedure designed to allow selective recovery of only such trimeric complexes of the invention that exhibit affinity for the corresponding substances (A, B and C in the illustration). Said affinity purification tag can be fused directly in-line or, alternatively, fused to the monomer polypeptide via a cleavable linker, i.e., a peptide segment containing an amino acid sequence that is specifically cleavable by enzymatic or chemical means (i.e., a recognition/cleavage site). In a particular embodiment, said cleavable linker comprises an amino acid sequence which is cleavable by a protease such as an enterokinase, Arg-C endoprotease, Glu-C endoprotease, Lys-C endoprotease, factor Xa, etc.; alternatively, in another particular embodiment, said cleavable linker comprises an amino acid sequence which is cleavable by a chemical reagent, such as, for example, cyanogen bromide which cleaves methionine residues, or any other suitable chemical reagent. The cleavable linker is useful if subsequent removal of the affinity purification tags is desirable.

In a particular embodiment, when the heterologous moiety is a peptide, said hetrologous moiety is preferably covalently linked to the XVTSE by via a peptide bond to the N- or C-terminus of the XVTSE peptide chain.

In a particular embodiment, said monomer polypeptide is directly covalently linked to said heterologous moiety. However, in another particular embodiment, said monomer polypeptide is not directly covalently linked to said heterologous moiety but it is linked through a spacer, i.e., an inert connecting or linking peptide of suitable length and character, between the monomer polypeptide and the heterologous moiety [i.e., forming a "monomer polypeptide-spacer-heterologous moiety" structure], or, alternatively, between the heterologous moiety and the monomer polypeptide [i.e., forming a "heterologous moiety-spacer-monomer polypeptide" structure]. In general, said spacer acts as a hinge region between said domains, allowing them to move independently from one another while maintaining the three-dimensional form of the individual domains. In this sense, a preferred spacer would be a hinge region characterized by a structural ductility or flexibility allowing this movement. The length of the spacer can vary; typically, the number of amino acids in the spacer is 100 or less amino acids, preferably 50 or less amino acids, more preferably 40 or less amino acids, still more preferably, 30 or less amino acids, or even more preferably 20 or less amino acids.

Illustrative, non-limitative examples of spacers include a polyglycine linker; amino acid sequences such as: SGGTSGSTSGTGST (SEQ ID NO: 3), AGSSTGSSTGPGSTT (SEQ ID NO: 4), GGSGGAP (SEQ ID NO: 5), GGGVEGGG (SEQ ID NO: 6), etc. In a particular embodiment, said spacer is a peptide having structural flexibility (i.e., a flexible linking peptide or "flexible linker") and comprises 2 or more amino acids selected from the group consisting of glycine, serine, alanine and threonine. In another particular embodiment, the spacer is a peptide containing repeats of amino acid residues, particularly Gly and Ser, or any other suitable repeats of amino acid residues. Virtually any flexible linker can be used as spacer according to this invention. Illustrative examples of flexible linkers include amino acid sequences such as Gly-Ser-Pro-Gly (GSPG; SEQ ID NO: 7), Ala-Ala-Ala-Gly-Gly-Ser-Gly-Gly-Ser-Ser-Gly-Ser-Arg (AAAGGSGGSSGSR; SEQ ID NO: 8) or Gly-Ser-Gly-Ser-Gly-Ser-Gly-Ser (Gly-Ser)4 (GSGSGSGS; SEQ ID NO: 9) . Nevertheless, in a particular embodiment said spacer is a flexible linker comprising the amino acid sequence Ala-Asn-Ser-Gly-Ala-Gly-Gly-Ser-Gly-Gly-Ser-Ser-Gly-Ser-Asp-Gly-Ala-Ser-Gly-Ser-Arg (ANSGAGGSGGSSGSDGASGSR; SEQ ID NO: 10).

Alternatively, a suitable spacer can be based on the sequence of 10 amino acid residues of the upper hinge region of murine IgG3; said peptide (PKPSTPPGSS, SEQ ID NO: 11) has been used for the production of dimerized antibodies by means of a coiled coil (Pack P. and Pluckthun, A., 1992, Biochemistry 31:1579-1584) and can be useful as a spacer peptide according to the present invention. Even more preferably, it can be a corresponding sequence of the upper hinge region of human IgG3 or other human Ig subclasses (IgG1, IgG2, IgG4, IgM and IgA). The sequences of human Igs are not expected to be immunogenic in human beings. Additional spacers that can be used in the instant invention include the peptides of sequences: APAETKAEPMT (SEQ ID NO: 12), GAP, AAA or AAALE (SEQ ID NO: 13).

In a particular embodiment, the monomer polypeptide is covalently linked to only one heterologous moiety, preferably, said heterologous moiety is covalently linked to only one end of said monomer polypeptide. In this case, the heterologous moiety can be linked by any of the above mentioned means, e.g., via a peptide bond to the amino-terminal end (N-terminus) or, alternatively, to the carboxyl-terminal end (C-terminus) of said monomer polypeptide comprising the XVTES peptide chain. Thus, in a more particular embodiment, the monomer polypeptide is linked to only one end of the heterologous moiety and said heterologous moiety is linked to the N-terminus of said monomer polypeptide (this type of monomer polypeptide can be designated as "N-terminal monomer polypeptide" and represented as "HM-XVTSE", wherein HM means heterologous moiety and XVTSE is that previously defined). In another particular embodiment, the monomer polypeptide is linked to only one end of the heterologous moiety and said heterologous moiety is linked to the C-terminus of said monomer polypeptide (this type of monomer polypeptide can be designated as "C-terminal monomer polypeptide" and represented as "XVTSE-HM", wherein HM means heterologous moiety and XVTSE is that previously defined).

In another particular embodiment, the monomer polypeptide is covalently linked to two heterologous moieties, preferably each heterologous moiety is covalently linked to just one of the ends of the monomer polypeptide by any of the previously mentioned methods (i.e., via a peptide bond to the N- or C-terminus of the XVTSE peptide chain, etc.). Thus, according to this particular embodiment, one of the heterologous moieties is linked to the N-terminus and the other one is linked to the C-terminus of said monomer polypeptide comprising the XVTES peptide chain, whereas in another specific embodiment, both heterologous moieties are linked each other and linked to just one terminus (N-terminus or C-terminus) of said monomer polypeptide comprising the XVTES peptide chain.

Thus, in said specific embodiment, a first heterologous moiety is linked to the N-terminus of said monomer polypeptide and a second heterologous moiety is linked to the C-terminus of said monomer polypeptide (this type of monomer polypeptide can be designated as N-/C-terminal monomer polypeptide and represented as "HM1-XVTSE-HM2", wherein HM1 means a first heterologous moiety, HM2 means a second heterologous moiety and XVTSE is that previously defined). Said first and second heterologous moieties can be equal or different. This particular embodiment, related to a monomer polypeptide comprising two heterologous moieties which are linked via peptide bonds to the N- and C-terminus, respectively, (i.e., N-/C-terminal monomer polypeptide) constitutes an interesting embodiment of the invention. This approach introduces a number of possibilities in terms of, for example, linking larger entities with oligomers of the invention by having specific activities coupled to each end of the monomers.

In another specific embodiment, a first heterologous moiety (HM1) is linked to a second heterologous moiety (HM2), in any order (HM1-HM2 or HM2-HM1), and also linked to only one terminus (i.e., the N-terminus or the C-terminus) of the monomer polypeptide thus forming, e.g., an "in-line" or "tandem" structure, linked to just one of the terminus of the monomer polypeptide, e.g., to the N-terminus, i.e., "HM1-HM2-XVTSE", "HM2-HM1-XVTSE", or to the C-terminus "XVTSE-HM1-HM2", or "XVTSE-HM2-HM1" of the monomer polypeptide. Said first and second heterologous moieties can be equal or different. Preferably, in said "in-line" or "tandem" structures the heterologous moieties are linked each other by means of a spacer; in a particular embodiment, said spacer is flexible linking peptide or a "flexible linker" as defined above.

In a specific embodiment, at least one of the heterologous moieties is linked to the N-terminus and at least one of the heterologous moieties is linked to the C-terminus of said monomer polypeptide comprising the XVTES peptide chain, whereas in another specific embodiment, all the heterologous moieties are linked each other and linked to just one terminus (N-terminus or C-terminus) of said monomer polypeptide comprising the XVTES peptide chain.

Thus, in a specific embodiment, at least one heterologous moiety is linked to the N-terminus of said monomer polypeptide and at least one heterologous moiety is linked to the C-terminus of said monomer polypeptide to render a monomer polypeptide; this type of monomer polypeptide can be represented as "(HM1)n-XVTES-(HM2)m", wherein the total number of heterologous moieties (n+m) is at least 3, each HM1 is, independently, equal or different to other HM1, and each HM2 is, independently, equal or different to other HM2. Further, each HM1 can be equal or different to each HM2. Illustrative, non-limitative, examples of this type of monomer polypeptide include monomer polypeptides in which one or more heterologous moieties are linked to the N-terminus of said monomer polypeptide and one or more heterologous moieties are linked to the C-terminus of said monomer polypeptide wherein the number of heterologous moieties is equal or higher than three.

As discussed above, the heterologous moieties can be directly linked each other, or, alternatively, in a particular embodiment, they can be linked though a spacer forming a "HM-spacer-HM" structure. The particulars or said spacer have been previously mentioned.

According to the instant invention, the trimeric complex of the invention comprises three monomer polypeptides, wherein (i) each of said monomer polypeptides comprises a XVTSE and (ii) at least one of said monomer polypeptides is covalently linked to at least one heterologous moiety.

Thus, in a particular embodiment, the trimeric complex of the invention comprises three monomer polypeptides, wherein (i) each of said monomer polypeptides comprises a XVTSE and (ii) only one of said monomer polypeptides is covalently linked to at least one heterologous moiety. According to this particular embodiment, only one of the monomer polypeptides which constitute the trimeric complex of the invention is covalently linked to at least one heterologous moiety. In a particular embodiment, said monomer polypeptide is covalently linked to one heterologous moiety. In another particular embodiment, said monomer polypeptide is covalently linked to two or more, e.g., 2, 3, 4 or even more, heterologous moieties; said heterologous moieties can be equal or different each other. The heterologous moiety o moieties can be linked to the N-terminus, or to the C-terminus, of the monomer polypeptide which comprises the XVTSE peptide chain. If the monomer polypeptide is covalently linked to two or more heterologous moieties, said heterologous moieties can be linked to the N-terminus, or to the C-terminus, of the monomer polypeptide which comprises the XVTSE peptide chain, or alternatively, at least one heterologous moiety can be linked to the N-terminus of the monomer polypeptide and at least one heterologous moiety can be linked to the C-terminus of the monomer polypeptide.

Illustrative, non-limitative, examples of this embodiment of the trimeric complex of the invention wherein only one monomer polypeptide is linked to at least one heterologous moiety include trimeric complexes of the invention in which:
- at least one heterologous moiety is linked at the N-terminus of the monomer polypeptide; or, alternatively,
- at least one heterologous moiety is linked at the C-terminus of the monomer polypeptide; or, alternatively,
- at least one heterologous moiety is linked at the N-terminus of the monomer polypeptide and at least one heterologous moiety is linked at the C-terminus of the monomer polypeptide.

In another particular embodiment, the trimeric complex of the invention comprises three monomer polypeptides, wherein (i) each of said monomer polypeptides comprises a XVTSE and (ii) each one of two of said monomer polypeptides is covalently linked to at least one heterologous moiety. According to this particular embodiment, each one of two of the three monomer polypeptides which constitute the trimeric complex of the invention, independently, is covalently linked to at least one heterologous moiety. Thus, according to this particular embodiment, the trimeric complex of the invention comprises a first monomer polypeptide covalently linked to at least one heterologous moiety and a second monomer polypeptide covalently linked to at least one heterologous moiety, wherein the heterologous moieties linked to each of said first and second monomer polypeptide can be equal or different. Further, as discussed above, said heterologous moiety or moieties can be linked to the N-terminus, or to the C-terminus, of the monomer polypeptide which comprises the XVTSE peptide chain, or alternatively, if any of the monomer polypeptides is covalently linked to two or more heterologous moieties, said heterologous moieties can be linked to the N-terminus, or to the C-terminus, of the monomer polypeptide which comprises the XVTSE peptide chain, or alternatively, at least one heterologous moiety can be linked to the N-terminus of the monomer polypeptide and at least one heterologous moiety can be linked to the C-terminus of the monomer polypeptide. The skilled person in the art will recognize that any combination of heterologous moieties can be present in this particular embodiment; i.e., in a specific embodiment, for example, a first monomer polypeptide can be linked to one or more heterologous moieties and a second monomer polypeptide can be linked to one or more heterologous moieties. In any case, said heterologous moiety or moieties can be linked to just one of the terminus (N- or C-terminus) of the monomer polypeptide or, alternatively, if any of the monomer polypeptide is covalently linked to two or more heterologous moieties, said heterologous moieties can be linked to the N-terminus, or to the C-terminus, of the monomer polypeptide which comprises the XVTSE peptide chain, or alternatively, at least one heterologous moiety can be linked to the N-terminus of the monomer polypeptide and at least one heterologous moiety can be linked to the C-terminus of the monomer polypeptide.

Illustrative, non-limitative, examples of this embodiment of the trimeric complex of the invention wherein each one of only two monomer polypeptides are linked to at least one heterologous moiety include trimeric complexes of the invention in which:
- at least one heterologous moiety is linked at the N-terminus of a first monomer polypeptide and at least one heterologous moiety is linked at the N-terminus of a second monomer polypeptide; or, alternatively,
- at least one heterologous moiety is linked at the C-terminus of a first monomer polypeptide and at least one heterologous moiety is linked at the C-terminus of a second monomer polypeptide; or, alternatively,
- at least one heterologous moiety is linked at the N-terminus of a first monomer polypeptide and at least one heterologous moiety is linked at the C-terminus of a second monomer polypeptide; or, alternatively,
- at least one heterologous moiety is linked at the N-terminus of a first monomer polypeptide and at least one heterologous moiety is linked at the C-terminus of said first monomer polypeptide, and at least one heterologous moiety is linked at the N-terminus of a second monomer polypeptide; or, alternatively,
- at least one heterologous moiety is linked at the N-terminus of a first monomer polypeptide and at least one heterologous moiety is linked at the C-terminus of said first monomer polypeptide, and at least one heterologous moiety is linked at the C-terminus of a second monomer polypeptide; or, alternatively,
- at least one heterologous moiety is linked at the N-terminus of a first monomer polypeptide and at least one heterologous moiety is linked at the C-terminus of said first monomer polypeptide, and at least one heterologous moiety is linked at the N-terminus of a second monomer polypeptide and at least one heterologous moiety is linked at the C-terminus of said second monomer polypeptide.

In another particular embodiment, the trimeric complex of the invention comprises three monomer polypeptides, wherein (i) each of said monomer polypeptides comprises a XVTSE and (ii) each one of the three monomer polypeptides is covalently linked to at least one heterologous moiety. According to this particular embodiment, each one of the three monomer polypeptides which constitute the trimeric complex of the invention, independently, is covalently linked to at least one heterologous moiety. Thus, according to this particular embodiment, the trimeric complex of the invention comprises a first monomer polypeptide covalently linked to at least one heterologous moiety, a second monomer polypeptide covalently linked to at least one heterologous moiety, and a third monomer polypeptide covalently linked to at least one heterologous moiety, wherein the heterologous moieties linked to each of said first, second and third monomer polypeptide can be equal or different each other, e.g., all the three heterologous moieties can be equal, or two of the three heterologous moieties can be equal each other and different to the other one, or, alternatively, all the three heterologous moieties can be different each other. The skilled person in the art will recognize that any combination of heterologous moieties can be present in this particular embodiment; i.e., in a specific embodiment, for example, a first monomer polypeptide can be linked to one or more heterologous moieties, the second monomer polypeptide can be linked to one or more heterologous moieties and the third monomer polypeptide can be linked to one or more heterologous moieties. In any case, said heterologous moiety or moieties can be linked to just one of the terminus (N- or C-terminus) of the monomer polypeptide or, alternatively, if any of the monomer polypeptide is covalently linked to two or more heterologous moieties, said heterologous moieties can be linked to the N-terminus, or to the C-terminus, of the monomer polypeptide which comprises the XVTSE peptide chain, or alternatively, at least one heterologous moiety can be linked to the N-terminus of the monomer polypeptide and at least one heterologous moiety can be linked to the C-terminus of the monomer polypeptide.

Illustrative, non-limitative, examples of this embodiment of the trimeric complex of the invention wherein each one of the monomer polypeptides are linked to at least one heterologous moiety include trimeric complexes of the invention in which:
- at least one heterologous moiety is linked at the N-terminus of a first monomer polypeptide, at least one heterologous moiety is linked at the N-terminus of a second monomer polypeptide and at least one heterologous moiety is linked at the N-terminus of a third monomer polypeptide; or, alternatively,
- at least one heterologous moiety is linked at the C-terminus of a first monomer polypeptide, at least one heterologous moiety is linked at the C-terminus of a second monomer polypeptide and at least one heterologous moiety is linked at the C-terminus of a third monomer polypeptide; or, alternatively,
- at least one heterologous moiety is linked at the N-terminus of a first monomer polypeptide, at least one heterologous moiety is linked at the C-terminus of a second monomer polypeptide and at least one heterologous moiety is linked at the N-terminus (or, alternatively, at the C-terminus) of a third monomer polypeptide; or, alternatively,
- at least one heterologous moiety is linked at the C-terminus of a first monomer polypeptide, at least one heterologous moiety is linked at the N-terminus of a second monomer polypeptide and at least one heterologous moiety is linked at the C-terminus (or, alternatively, at the N-terminus) of a third monomer polypeptide; or, alternatively,
- at least one heterologous moiety is linked at the N-terminus of a first monomer polypeptide and at least one heterologous moiety is linked at the C-terminus of said first monomer polypeptide, at least one heterologous moiety is linked at the N-terminus of a second monomer polypeptide and at least one heterologous moiety is linked at the N-terminus of a third monomer polypeptide; or, alternatively,
- at least one heterologous moiety is linked at the N-terminus of a first monomer polypeptide and at least one heterologous moiety is linked at the C-terminus of said first monomer polypeptide, at least one heterologous moiety is linked at the C-terminus of a second monomer polypeptide and at least one heterologous moiety is linked at the C-terminus of a third monomer polypeptide; or, alternatively,
- at least one heterologous moiety is linked at the N-terminus of a first monomer polypeptide and at least one heterologous moiety is linked at the C-terminus of said first monomer polypeptide, at least one heterologous moiety is linked at the N-terminus of a second monomer polypeptide and at least one heterologous moiety is linked at the C-terminus of a third monomer polypeptide; or, alternatively,
- at least one heterologous moiety is linked at the N-terminus of a first monomer polypeptide and at least one heterologous moiety is linked at the C-terminus of said first monomer polypeptide, at least one heterologous moiety is linked at the N-terminus of a second monomer polypeptide and at least one heterologous moiety is linked at the C-terminus of said second monomer polypeptide and at least one heterologous moiety is linked at the N-terminus (or, alternatively, at the C-terminus) of a third monomer polypeptide; or, alternatively,
- at least one heterologous moiety is linked at the N-terminus of a first monomer polypeptide and at least one heterologous moiety is linked at the C-terminus of said first monomer polypeptide, at least one heterologous moiety is linked at the N-terminus of a second monomer polypeptide and at least one heterologous moiety is linked at the C-terminus of said second monomer polypeptide, and at least one heterologous moiety is linked at the N-terminus of a third monomer polypeptide and at least one heterologous moiety is linked at the C-terminus of said third monomer polypeptide.

Thus, as disclosed above, the trimeric complex of the invention may be a homotrimer (i.e., all the monomer polypeptides are equal each other) or a heterotrimer (i.e., at least one of the monomer polypeptides is different from the other two monomer polypeptides).

In a particular embodiment, when the heterologous moiety is an antibody or a fragment thereof, the trimeric complex of the invention may be monospecific (e.g., when the heterologous moiety (e.g., antibody or fragment thereof) in each one of the three monomer polypeptides which constitute the trimeric complex of the invention is the same, i.e., it is specific for an antigen), bispecific (e.g., when one of the heterologous moieties (e.g., an antibody or fragment thereof recognizes a first antigen) in one of the monomer polypeptides which constitute the trimeric complex of the invention is different from another heterologous moiety (e.g., an antibody or fragment thereof which recognizes a second antigen - wherein said second antigen is different from said first antigen) in the same or different monomer polypeptide), or even, the specificity of the trimeric complex of the invention may be higher, (e.g., when one of the heterologous moieties (e.g., an antibody or fragment thereof recognizes a first antigen) in one of the monomer polypeptides which constitute the trimeric complex of the invention is different from another heterologous moiety (e.g., an antibody or fragment thereof which recognizes a second (different) antigen) in the same or different monomer polypeptide) and different from another heterologous moiety (e.g., an antibody or fragment thereof which recognizes a third (different) antigen) in the same or different monomer polypeptide).

Therefore, the trimeric complex of the invention constitutes an enormously flexible platform for different applications, including protein engineering.

Effectively, a particularly interesting embodiment of the invention is the possibility of designing oriented molecular assemblies, where one or more functional entities are located N-terminally to the XVTSE and one or more functional entities are located C-terminally to said trimerizing element (XVTSE). Such types of design may be particularly advantageous where a certain relative ratio is desired among the different functional entities included in a specific molecular unit. Such type of design may in addition be used if one or more functional entities for either structural or functional reasons appear incompatible within the same construct. Such may be the case, e.g., if one or more of the functional entities are expressed by large or bulky protein domains which for steric reasons might prevent formation of the trimeric molecular unit due to sterical constraints.

Further, the possibility of constructing bi-polar three-way fusion proteins in which one functionality is placed at the N-terminus of the XVTSE and a different functionality is placed at the C-terminus of said XVTSE is additionally advantageous in applications where large spatial separation between the two functionalities are desirable for optimal function. Examples of such application are, e.g., the deployment of binding domains (e.g. antibody-derived binding modules) for recognition and binding to binding sites located at or close to large structures like cell membranes in cases where it is advantageous to allow for binding of the other end of the trimeriztrimerized molecule to a different, but also bulky target.

Hence, as discussed above, the trimeric complex of the invention may be used to join, e.g., bulky surfaces by said complex comprising at least one heterologous moiety which is positioned at the N-terminus of the XVTSE and at least one heterologous moiety which is positioned at the C-terminus of said XVTSE. The two heterologous moieties can be either part of the same monomer polypeptide or part of two separate monomer polypeptides.

The XVTSE will, essentially indiscriminately, form homo- and hetero-trimers with any molecule that also contains said trimerization module. For some applications, it may be advantageous to have available specially engineered derivatives of the XVTSE, which have been reengineered to disallow homo-trimer formation and hence only allow hetero-trimerization. Thus, an important embodiment of the monomer polypeptide which constitutes the trimeric complex of the invention is constructed/reengineered so as to disfavour formation of complexes between identical XVTSEs; this also has the implication that said monomer polypeptides can advantageously be designed so as to disfavour formation of trimers including two monomer polypeptides having identical XVTSEs. One way of disfavouring the formation of homo-trimerization would be by "knobs into holes" mutagenesis.

The design/reengineering may be accomplished by introduction of amino acid substitution at sites in the monomer polypeptide intimately involved in the formation and stability of the trimer and, simultaneously, in a different construct introduce a compensatory amino acid substitution, all in all removing symmetry between individual monomer components of the triple helical structure so that the structural complementarity profile only allows the formation of hetero-trimers, but is incompatible with some or each of the homotrimer species.

The trimeric complex of the invention may be prepared by methods generally known in the art, based, for example, techniques of recombinant protein production. Hence the invention also relates to a method of preparing the trimeric complex of the invention, the method comprising isolating the trimeric complex of the invention from a culture comprising a host cell which carries and expresses a nucleic acid fragment which encodes at least one of the monomer polypeptides of the trimeric complex of the invention, and, optionally, subjecting the trimeric complex of the invention to further processing.

The nucleic acid fragment which is mentioned above, hereinafter referred to as the nucleic acid of the invention, is also a part of the invention and is defined as a nucleic acid fragment in isolated form which encodes a XVTSE as defined herein or which encodes the polypeptide part of a monomer polypeptide according to the invention. In a particular, embodiment, the sequence of the nucleic acid of the invention comprises, or consists of, the nucleotide sequence shown in SEQ ID NO: 2: gtgaccgccttcagcaacatggacgacatgctgcagaaggcccacctggtcatcgagggc 
accttcatctacctgcgggacagcaccgagttcttcatcagagtgcgggacggctggaag
aagctgcagctgggcgagctgatccccatccccgccgatagccctccacctcctgccctg
agcagcaaccct
 which corresponds to the N-terminus region of the human collagen XV NC1 domain.

In a particular embodiment, the nucleic acid of the invention comprises the nucleotide sequence encoding a XVTSE as defined herein.

In another particular embodiment, the nucleic acid of the invention comprises the nucleotide sequence encoding the polypeptide part of a monomer polypeptide which is present in the trimeric complex of the invention. The polypeptide part of said monomer polypeptide comprises the XVTSE and the heterologous moiety when the heterologous moiety is a peptide. As it has been previously mentioned, the monomer polypeptide may be fused to one or more heterologous moieties said one or more heterologous moieties being fused to one or both ends of the monomer polypeptide; thus, when said heterologous moieties are peptides, the nucleic acid of the invention comprises the nucleotide sequence encoding the XVTSE and the nucleotide sequence or sequences encoding said heterologous moiety or moieties. In that case, the 5' end of the nucleotide sequence encoding said heterologous moiety can be fused to the 3' end of the nucleotide sequence encoding the XVTSE, or, alternatively, the 3' end of the nucleotide sequence encoding said heterologous moiety can be fused to the 5' end of the nucleotide sequence encoding the XVTSE. Said nucleotide sequences can be operatively linked so that each sequence is correctly expressed by just one promoter or, alternatively, each nucleotide sequence is under the control of independent promoters. Said promoter(s) can be inducible or constitutive. The nucleic acid of the invention may include, if desired, operatively linked, the nucleotide sequence encoding the spacer between the monomer polypeptide and the heterologous moiety and/or, the nucleotide sequence encoding the cleavable linker between the monomer polypeptide and the tag (heterologous moiety).

The nucleic acid of the invention can be prepared by traditional genetic engineering techniques.

The above mentioned host cell, hereinafter referred to as the host cell of the invention, which is also a part of the invention, can be prepared by traditional genetic engineering techniques which comprise inserting the nucleic acid of the invention into a suitable expression vector, transforming a suitable host cell with the vector, and culturing the host cell under conditions allowing expression of the XVTSE or the polypeptide part of the monomer polypeptide of the invention. Said vector comprising the nucleic acid of the invention, hereinafter referred to as the vector of the invention, is also a part of the invention. The nucleic acid of the invention may be placed under the control of a suitable promoter which may be inducible or a constitutive promoter. Depending on the expression system, the polypeptide may be recovered from the extracellular phase, the periplasm or from the cytoplasm of the host cell.

Suitable vector systems and host cells are well-known in the art as evidenced by the vast amount of literature and materials available to the skilled person. Since the present invention also relates to the use of the nucleic acid of the invention in the construction of vectors and in host cells, the following provides a general discussion relating to such use and the particular considerations in practising this aspect of the invention.

In general, of course, prokaryotes are preferred for the initial cloning of the nucleic acid of the invention and constructing the vector of the invention. For example, in addition to the particular strains mentioned in the more specific disclosure below, one may mention by way of example, strains such as *E. coli* K12 strain 294 (ATCC No. 31446), *E. coli* B, and *E. coli* X 1776 (ATCC No. 31537). These examples are, of course, intended to be illustrative rather than limiting.

Prokaryotes can be also utilized for expression, since efficient purification and protein refolding strategies are available. The aforementioned strains, as well as *E. coli* W3110 (F-, lambda-, prototrophic, ATCC No. 273325), bacilli such as *Bacillus subtilis,* or other enterobacteriaceae such as *Salmonella typhimurium* or *Serratia marcesans,* and various *Pseudomonas* species may be used.

In general, plasmid vectors containing replicon and control sequences which are derived from species compatible with the host cell are used in connection with these hosts. The vector ordinarily carries a replication site, as well as marking sequences which are capable of providing phenotypic selection in transformed cells. For example, *E. coli* is typically transformed using pBR322, a plasmid derived from an *E. coli* species. The pBR322 plasmid contains genes for ampicillin and tetracycline resistance and thus provides easy means for identifying transformed cells. The pBR322 plasmid, or other microbial plasmid or phage must also contain, or be modified to contain, promoters which can be used by the microorganism for expression.

Those promoters most commonly used in recombinant DNA construction include the B-lactamase (penicillinase) and lactose promoter systems and a tryptophan (trp) promoter system (EP 36776). While these are the most commonly used, other microbial promoters have been discovered and utilized, and details concerning their nucleotide sequences have been published, enabling a skilled worker to ligate them functionally with plasmid vectors. Certain genes from prokaryotes may be expressed efficiently in *E. coli* from their own promoter sequences, precluding the need for addition of another promoter by artificial means.

In addition to prokaryotes, eukaryotic microbes, such as yeast cultures may also be used. *Saccharomyces cerevisiase,* or common baker's yeast is the most commonly used among eukaryotic microorganisms, although a number of other strains are commonly available. For expression in *Saccharomyces,* the plasmid YRp7, for example, is commonly used. This plasmid already contains the *trpl* gene which provides a selection marker for a mutant strain of yeast lacking the ability to grow in tryptophan for example ATCC No. 44076 or PEP4-1 (Jones, 1977, Genetics, 85:23-33). The presence of the *trpl* lesion as a characteristic of the yeast host cell genome then provides an effective environment for detecting transformation by growth in the absence of tryptophan.

Suitable promoting sequences in yeast vectors include the promoters for 3-phosphoglycerate kinase or other glycolytic enzymes, such as enolase, glyceraldehyde-3-phosphate dehydrogenase, hexokinase, pyruvate decarboxylase, phosphofructokinase, glucose-6-phosphate isomerase, 3-phosphoglycerate mutase, pyruvate kinase, triosephosphate isomerase, phosphoglucose isomerase, and glucokinase. In constructing suitable expression plasmids, the termination sequences associated with these genes are also ligated into the expression vector 3' of the sequence desired to be expressed to provide polyadenylation of the mRNA and termination.

Other promoters, which have the additional advantage of transcription controlled by growth conditions are the promoter region for alcohol dehydrogenase-2, isocytochrome C, acid phosphatase, degradative enzymes associated with nitrogen metabolism, and the aforementioned glyceraldehyde-3-phosphate dehydrogenase, and enzymes responsible for maltose and galactose utilization. Any plasmid vector containing a yeast-compatible promoter, origin of replication and termination sequences is suitable.

In addition to microorganisms, cultures of cells derived from multicellular organisms may also be used as hosts. In principle, any such cell culture is workable, whether from vertebrate or invertebrate culture. However, interest has been greatest in vertebrate cells, and propagation of vertebrate in culture (tissue culture) has become a routine procedure in recent years. Examples of such useful host cell lines are VERO and HeLa cells, Chinese hamster ovary (CHO) cell lines, and W138, BHK, COS-7, Human Embryonic Kidney (HEK) 293 and MDCK cell lines.

Expression vectors for such cells ordinarily include (if necessary) an origin of replication, a promoter located in front of the gene to be expressed, along with any necessary ribosome binding sites, RNA splice sites, polyadenylation site, and transcriptional terminator sequences.

For use in mammalian cells, the control functions on the expression vectors are often provided by viral material; for example, commonly used promoters are derived from polyoma, Adenovirus 2, cytomegalovirus (CMV) and most frequently Simian Virus 40 (SV40). The early and late promoters of SV40 virus are particularly useful because both are obtained easily from the virus as a fragment which also contains the SV40 viral origin of replication. Smaller or larger SV40 fragments may also be used, provided there is included the approximately 250 bp sequence extending from the *HindIII* site toward the *BglI* site located in the viral origin of replication. Further, it is also possible, and often desirable, to utilize promoter or control sequences normally associated with the desired gene sequence, provided such control sequences are compatible with the host cell systems.

An origin of replication may be provided either by construction of the vector to include an exogenous origin, such as may be derived from SV40 or other viral (e.g., polyoma, adeno, etc.) or may be provided by the host cell chromosomal replication mechanism. If the vector is integrated into the host cell chromosome, the latter is often sufficient.

Upon production of the monomer polypeptide which constitutes the trimeric complex of the invention, it may be necessary to process the polypeptides further, e.g. by introducing non-proteinaceous functions in the polypeptide, by subjecting the material to suitable refolding conditions (e.g. by using the generally applicable strategies suggested in WO 94/18227), or by cleaving off undesired peptide moieties of the monomer (e.g. expression enhancing peptide fragments which are undesired in the end product).

In the light of the above discussion, the methods for recombinantly producing said trimeric complex of the invention or monomer polypeptide according to the invention are also a part of the invention, as are the vectors carrying and/or being capable of replicating the nucleic acid of the invention in a host cell or in a cell-line. According to the invention the expression vector can be, e.g., a virus, a plasmid, a cosmid, a minichromosome, or a phage. Especially interesting are vectors which are integrated in the host cell/cell line genome after introduction in the host.

Another aspect of the invention are transformed cells (i.e., the host cell of the invention), useful in the above-described methods, carrying and capable of replicating the nucleic acid of the invention; the host cell can be a microorganism such as a bacterium, a yeast, or a protozoan, or a cell derived from a multicellular organism such as a fungus, an insect cell, a plant cell, or a mammalian cell. Especially interesting are cells from the bacterial species *Escherichia, Bacillus* and *Salmonella,* and a preferred bacterium is *E. coli.*

Yet another aspect of the invention relates to a stable cell line producing the monomer polypeptide according to the invention or the polypeptide part thereof, and preferably the cell line carries and expresses a nucleic acid of the invention. Especially interesting are cells derived from the mammalian cell lines HEK and CHO.

As it is evident for the skilled person in the art, the trimer complex of the invention may be a homotrimer (i.e., all the three monomer polypeptides are identical) or a heterotrimer (i.e., at least one of the three monomer polypeptides is different from the others).

When the trimer complex of the invention is a homotrimer, the method for recombinantly producing said homotrimer comprises inserting the nucleic acid of the invention into a suitable expression vector, transforming a suitable host cell with the vector, and culturing the host cell under conditions allowing expression of the monomer polypeptide according to the invention and trimerization thereof In this case, only one nucleic acid of the invention has to be prepared, said nucleic acid of the invention comprising the nucleotide sequence encoding the XVTSE and the nucleotide sequence encoding the polypeptide part of the heterologous moiety. The considerations applying to further processing mentioned above apply to this method also.

When the trimer complex of the invention is a heterotrimer, said heterotrimer may comprise (i) only one monomer polypeptide different from the other two monomer polypeptides, this two monomer polypeptides being identical each other, or, alternatively, (ii) three different monomer polypeptides.

In the first case, when the trimer complex of the invention is a heterotrimer wherein only one monomer polypeptide (MP1) is different from the other two monomer polypeptides (MP2), the method for recombinantly producing said heterotrimer comprises inserting a first nucleic acid of the invention comprising the nucleotide sequence encoding said monomer polypeptide (MP1) into a suitable expression vector, and a second nucleic acid of the invention comprising the nucleotide sequence encoding the other monomer polypeptide (MP2) into a suitable expression vector, transforming (co-transfecting) a suitable host cell with said vectors, and culturing the host cell under conditions allowing expression of the monomer polypeptides according to the invention and trimerization thereof to render the heterotrimer. In this case, two nucleic acids of the invention have to be prepared, one of them comprising the nucleotide sequence encoding a monomer polypeptide (e.g., Mop1) and the other one encoding the other monomer polypeptide (MP2). The considerations applying to further processing mentioned above apply to this method also.

In the second case, when the trimer complex of the invention is a heterotrimer wherein all the three monomer polypeptides are different each other (e.g., MP1, MP2 and MP3), the method for recombinantly producing said heterotrimer comprises inserting a first nucleic acid of the invention comprising the nucleotide sequence encoding monomer polypeptide 1 (MP1) into a suitable expression vector, inserting a second nucleic acid of the invention comprising the nucleotide sequence encoding monomer polypeptide 2 (MP2) into a suitable expression vector, and inserting a third nucleic acid of the invention comprising the nuclotide sequence encoding monomer polypeptide 3 (MP3) into a suitable expression vector, transforming (co-transfecting) a suitable host cell with said vectors, and culturing the host cell under conditions allowing expression of the monomer polypeptides according to the invention and trimerization thereof to render the heterotrimer. In this case, three nucleic acids of the invention have to be prepared, one of them comprising the nucleotide sequence encoding one monomer polypeptide (e.g., MP1), another encoding other monomer polypeptide (MP2), and another comprising the nucleotide sequence encoding another monomer polypeptide (MP3). The considerations applying to further processing mentioned above apply to this method also.

A very important aspect of the invention is the possibility of generating a system designed especially for the individual circumstances. The basic idea is that the artificial selection of heterologous moieties and optionally active components, and functional entities result in a unique system as will be further disclosed in the following.

Using the XVTSE as a vehicle for assembling monovalent scFv or Fab antibody fragments into oligomeric and multivalent entities offer design advantages also in terms of generating chimaeric artificial antibodies having desirable pharmacokinetic and pharmacodynamic properties. Small derivatives like monomeric scFv fragments or bivalent "minibodies" are rapidly cleared from the circulatory system, whereas native Igs have longer half-life. Conversely, small derivatives like scFv and minibodies exhibit better extravasation properties. It is therefore expected that antibodies of a desired specificity may be optimized for particular diagnostic or therapeutic needs by engineering the pharmacological properties, using the XVTSE as a vehicle for controlled oligomerization of e.g. scFv fragments.

One example of such engineering would be the requirements for delivering a high dose of an imaging or toxin-conjugated antibody to a tumor, while ensuring as low a systemic exposure or imaging background as possible. In such case a XVTSE-fused or conjugated scFv fragment could be designed to exhibit strong multivalent binding to the tumor and rapid clearance of excess fusion protein or conjugate from circulation.

Accordingly, in a further aspect, the present invention also relates to the use of the trimeric complex of the invention as a vehicle for assembled antibody fragments thus generating chimeric artificial antibodies having preselected pharmacokinetic and/or pharmacodynamic properties.

The use of specific delivery systems also play an important role in connection with the present invention in that such systems may be utilized with respect to different use of the present invention both with respect to the a more general therapeutic application and with respect to gene therapy. Examples of suitable drug delivery and targeting systems are disclosed in Nature 392 supp. (30 Apr. 1998).

Furthermore, a review of the imaging and therapeutic potential of a range of known antibody derivatives has been published [Holliger & Hudson, Nature Biotechnology, 2005, vol 23, 1126-1136; and Wu & Senter, Nature Biotechnology, 2005, vol 23, 1137-1146] In direct continuation of their conclusions it will be apparent that oligomerisation of antibody derivatives like scFv derivatives may extend current technology in the designer-antibody field in many important aspects, some of which will be elaborated below.

One of the well-known problems inherent to mouse monoclonal antibodies that have been "humanized" by grafting of the murine antigen combining site onto a human Ig framework is that antigenicity of the chimaeric product in human patients is often difficult to suppress entirely, resulting in sometimes life threatening immune reactions to the diagnostic or therapeutic humanized antibody product. The risks of such side-effects are expected to be much reduced if the designed antibody is assembled from purely human proteins or protein fragments. Since, in a particular embodiment, the XVTSE trimerization unit described herein is identical to a portion of human collagen XV that is already present in human plasma and tissue, there is good reason to expect that the XVTSE will not elicit an antigenic response in a human subject if it is introduced as a component of a chimaeric product that is not otherwise antigenic in humans.

Accordingly, in another aspect, the present invention relates to the use of a trimeric complex of the invention or a monomer polypeptide according to the present invention as a component of a chimaeric product having low antigenicity in humans relative to formulations comprising on or more components of non-human origin.

In connection with the technology for radiolabelling of antibody derivatives, again, oligomerisation using XVTSE offer more elegant solutions to problems associated with labelling, as the XVTSE offers the possibility to construct one or two of the XVTSE monomer units in a heterotrimeric complex to harbour the site carrying the label. Thus, in this format labelling may also be confined to the non-antibody part of the complex, leaving the antigen-binding module entirely unmodified, and the complex may furthermore be formulated "in the field" as and when needed.

In molecular trapping approaches, it is important to increase the antibody size above the renal threshold to retard clearance rates. Trivalent antibodies (a particular embodiment of the trimeric complex of the invention), or trivalent soluble truncated versions of membrane receptors (triple trap) would have an increased binding stoichiometry to soluble cytokines and growth factors. Coupled with slowed clearance rates, the greater avidity of trivalent antibodies might translate into a greater ability than their monovalent or bivalent counterparts to bind and sequester soluble molecules. This can also be applied to other pathogenic particles against which antibody antidotes have been generated. These include toxins, such as botulinum neurotoxin A or anthrax toxin, and viruses, such as hepatitis viruses or varicella-zoster virus, in which multimerization has been demonstrated to be needed for effective neutralization.

In many receptor-mediated signal transduction pathways signals are triggered by the clustering of receptor molecules on the cell membrane. The XVTSEs therefore have important applications in the study and exploitation of receptor signalling, especially for receptors that trimerize upon ligand binding such as those of the TNF family of receptors (eg. CD137, 0X40) as ligands may be presented as trimers by genetic fusion or conjugation to a XVTSE unit. This also has important application in phage display technologies for discovering new ligands and new receptors as the engineering of a XVTSE unit fused in-line to a candidate ligand molecule will allow the display of a hetero-trimeric phage coat protein, in which only one of the monomer units is linked to the phage coat protein. This may be accomplished by appropriate insertion of amber codons at the site of fusion of phage coat protein to the XVTSE-ligand segment of the three-way fusion protein encoded by the recombinant phage. In appropriate *E*. *coli* cells the presence of this amber codon will result in translation termination in the majority of read-throughs, and hence most of the fusion protein product secreted to the periplasmic compartment in the phage-infected bacterium will be soluble XVTSE-ligand fusion protein, whereas a minority of the fusion protein will also contain a phage protein module. The majority of trimers that will be generated will therefore contain, at most, one monomeric unit that will ensure integration (display) in the mature recombinant phage particle.

A further advantage of the display technology described above relates to the fact that it is specially useful for selection on the basis of a relatively low affinity because of the entropic benefit contribution obtained by the proximity of the three binding moieties in confined spatial arrangement. Accordingly, the present invention in an important aspect, also relates to protein library technology wherein the XVTSE's described above are utilized.

The trimerization of candidate recombinant ligands is especially important as, for many receptors, the intracellular signal is induced by receptor clustering, which is only brought about if the external ligand exhibits multivalent binding to the receptor, so as to bridge two or more receptor molecules.

As mentioned above, it is an important aspect of the invention that the trimeric complex of the invention or a monomer polypeptide according to the invention may be used as a component of a chimaeric product having low antigenicity in humans. As the monomer polypeptide can be of human origin it is believed that the antigenicity in humans is low relative to formulations comprising on or more components of non-human origin.

One primary use of a trimeric complex of the invention or a monomer polypeptide according to the invention is for delivering an imaging or toxin-conjugated or genetically fused antibody to a target such as a tumor, or use as a vehicle delivering a substance to a target cell or tissue, as a vehicle for assembling antibody fragments into oligomeric or multivalent entities for generating chimeric artificial antibodies having pre-selected pharmacokinetic and/or pharmacodynamic properties.

The substance in question being one or more selected from the group of heterologous moieties as well as a pharmaceutical. Also a labelled construct wherein the label is coupled to one or more of the XVTSE monomer units is within the scope of the invention.

As explained in detail previously, an important and surprising use of the trimeric complex of the invention or a monomer polypeptide according to the present invention is for protein library technology, such as phage display technology.

A further use according to invention includes the preparation and use of a pharmaceutical composition comprising the trimeric complex of the invention or a monomer polypeptide according to the invention and optionally a pharmaceutically acceptable excipient. The composition may be administered by a route selected from the group consisting of the intravenous route, the intra-arterial route, the intravitreal route, the transmembraneus route of the buccal, anal, vaginal or conjunctival tissue, the intranasal route, the pulmonary route, the transdermal route, the intramuscular route, the subcutaneous route, the intratechal route, the oral route, inoculation into tissue such as a tumor, or by an implant.

It is obvious from the disclosure of the present invention that the treating or preventing of a disease may be a further aspect comprising administering to the subject in need thereof an effective amount of a pharmaceutical composition referred to above.

As used in the conventional pharmaceutical field the present invention includes a method wherein the trimeric complex of the invention or a monomer polypeptide according to the invention is administered by a route selected from the group consisting of the intravenous route, the intra-arterial route, the transmembraneus route of the buccal, anal or vaginal tissue, intranasal route, intravitreal route, the pulmonary route, the transdermal route, intramuscular route, subcutaneous route, intratechal route, the oral route, inoculation into tissue such as a tumor, or by an implant.

Finally, the present invention is also related to the field of diagnosing as the skilled person would easily recognize that the XVTSE disclosed herein may also refer to a method for diagnosis comprising a trimeric complex of the invention together with a diagnosing component coupled thereon.

### EXAMPLE 1

### Improved stability of recombinant antibody-based molecules containing the trimerization region from the human collagen XV NCl domain

### I. MATERIAL & METHODS

### Reagents and antibodies

The monoclonal antibody (mAb) specific for human c-myc used was 9E10 (Abcam, Cambridge, UK). The IRDye 800 (fluorocrome)-conjugated goat anti-mouse IgG polyclonal antibody (Fc specific) was from Rockland Immunochemicals Inc (Gilbertsville, PA, USA). Laminin 111 extracted from the Engelbreth-Holm-Swarm (EHS) mouse tumor was from Becton Dickinson Labware (Bedford, MA, USA). Human and mouse sera, and proteases (Cathepsin L and elastase) were from Sigma-Aldrich Inc. (St. Louis, MO, USA).

### Cells and culture conditions

HEK-293 cells (human embryo kidney epithelia; CRL-1573) were cultured in Dulbecco's modified Eagle's medium (DMEM) supplemented with 10% (vol/vol) heat-inactivated Fetal Calf Serum (FCS) (all from Invitrogen, Carlsbad, CA) referred as to DMEM complete medium (DCM).

### Construction of expression vectors

To construct the pCR3.1-L36-hXVNC1ES- expression vector the N-terminal trimerization region from human collagen XV NC1 domain (hXVNC1ES-) [from nucleotide 1133 to nucleotide 1198] was synthesized by Geneart AG (Regensburg, Germany) and subcloned as NotIXbaI into the vector pCR3.1-L36 (Sanz, L. and Álvarez-Vallina, L. Antibody-based antiangiogenic cancer therapy (2005) Expert. Opin. Ther. Targets. 9, 1235-1245) containing the anti-laminin L36 single chain Fv (scFv) gene. The vector pCR3.1-L36-mXVIIINC1ES- containing the anti-laminin L36 scFv and the N-terminal trimerization region from murine collagen XVIII NC1 domain (mXVIIINC1ES-) was constructed as previously described [Sanchez-Arevalo, L. et al. Enhanced antiangiogenic therapy with antibody-collagen XVIII NC1 domain fusion proteins engineered to exploit matrix remodeling events (2006) Int. J. Cancer 119, 455-462].

### Cell transfections and purification of recombinant antibodies

HEK-293 cells were transfected with pCR3.1-L36-mXVIIINC1ES- or pCR3.1-L36-hXVNC1ES- expression vectors using Superfect (QIAGEN GmbH, Hilden, Germany), and selected in DCM supplemented with G418 (Geneticin) [500 µg/ml]. Supernatants from transient and stable transfected cell populations were analyzed for protein expression by ELISA, SDS-PAGE and Western blotting using anti-myc mAb. Stable transfected HEK-293 cells were used to collect serum-free conditioned media medium (approximately 1 liter), and loaded onto a HisTrap HP 1 ml column using an ÄKTA Prime plus system (GE Healthcare, Uppsala, Sweden). The purified antibodies were dialyzed against PBS, analyzed by SDS-PAGE under reducing conditions, and stored at -20°C.

### ELISA

The ability of recombinant antibody constructs to bind laminin was studied by ELISA as described (Sanz, L. et al. Generation of non-permissive basement membranes by anti-laminin antibody fragments produced by matrix-embedded gene-modified cells (2003) Cancer Immunol. Immunother. 52, 643-647).

### Serum stability

To determine whether recombinant antibody constructs remained functional in serum, 1 microgram of purified antibodies were incubated in 62% human or mouse serum at 37°C for up to 72 h. Samples were removed for analysis at 3 h, 24 h, 48h, and 72 h following the start of incubation and frozen until the entire study was completed. As a control, a second set of serum-exposed samples was frozen immediately to represent a zero time point. Recombinant antibodies were then tested for their capability to bind laminin by ELISA.

### Protease stability

To determine whether recombinant antibody constructs remained functional when incubated with different proteases, 1 microgram (µg) of purified antibodies were incubated in 0, 200, 300, and 400 ng of cathepsin L protesase, or in 0, 50, 100 and 200 ng of elastase protease at 37°C for 10 minutes. After incubation samples were tested for their capability to bind laminin by ELISA and its structural integrity were analyzed by Western blot.

### II. RESULTS

### Design and expression of recombinant antibodies constructs

The NC1 domains of collagens XVIII and XV have similar primary structures (Boudko SP et al. Crystal structure of human collagen XVIII trimerization domain: A novel collagen trimerization Fold. (2009) J Mol Biol. 392(3):787-802), which are organized into three different subdomains: an amino-terminal region potentially responsible for homotrimerization is followed by a protease-labile segment and the endostatin domain. The inventors have previously shown that fusion of the N-terminal trimerization region of the murine collagen XVIII NC1 domain (mXVIIINC1ES-) to the C-terminus of the L36 scFv fragment (L36-mXVIIINC1ES-) confers their natural oligomeric state to the fused antibody. The homo-trimeric molecules were isolated in functional active form from the cell culture supernatant of gene-modified 293 cells (Sanchez-Arevalo, L. et al. Enhanced antiangiogenic therapy with antibody-collagen XVIII NC1 domain fusion proteins engineered to exploit matrix remodeling events (2006) Int. J. Cancer 119, 455-462).

In this study, inventors have extended the concept by designing trimeric antibodies using the N-terminal trimerization region of the human collagen XV NC1 domain (hXVNC1ES-). Starting from the L36 scFv gene a new recombinant antibody was generated. The L36 scFv-hXVNC1ES- fusion protein was secreted as soluble functional protein in transfected human 293 cells (Figures 1A and 1B), and purified from conditioned medium by immobilized metal affinity chromatography. The purification scheme yielded antibodies that were >95% pure by SDS―PAGE, showing a single band of 39.7 kDa in reducing conditions.

### Antigen Binding Activity

The functionality of purified recombinant L36-hXVNC1ES- antibody was demonstrated by ELISA against plastic immobilized laminin (Figure 1C). These results demonstrated that the L36-hXVNC1ES- antibody recognized the cognate antigen as efficiently as the conventional L36-derived antibody, with the trimerization domain from mouse collagen XVIII.

### Structural characterization

L36-hXVNCIES- recombinant antibody was analyzed by analitycal centrifugation [(1 µg/ml in PBS) at 11,000 rpm, 20°C] and sedimentation velocity [(1 µg/ml in PBS) at 42,000 rpm, 20°C] to check their oligomeric and molecular weight. The results are shown in Figures 2 and 3, respectively. Sedimentation velocity showed a single peak with a mass of 117.6 kDa, and the mass distribution in a sedimentation equilibrium experiment could only be fitted to a trimer (not to a monomer or a dimer).

### Stability in vitro

The stability of engineered antibody fragments in serum is the critical parameter to determine their potential application *in vivo.* Therefore, inventors compared the functionality of both NC1 collagen derived constructs (L36- mXVIIINC1ES- and L36-hXVNC1ES-) after incubation in mouse or human serum at 37°C, for prolonged periods of time. As shown in Figure 4, the purified L36 antibody containing the collagen XV NC1 trimerization region [L36-hXVNC1ES-], had a significantly improved stability than the conventional L36 antibody containing the collagen XVIII NC1 trimerization region [L36-mXVIIINC1ES-] (77 and 90% vs. 45-59%, in human or mouse serum, respectively).

Furthermore, their structural integrity and functional stability were compared by incubation, for 10 minutes at 37°C, with different amounts of proteases (cathepsin L and elastase). As shown in Figures 5 and 6, L36-mXVIIINC1ES- construct was completely degraded in presence of both proteases and showed a residual activity of 20%. By contrast, L36-hXVNC1ES- construct retains their structural integrity and functionality up to 70% when incubated with 400 ng of cathepsin L and 30% when incubated with 200 ng of elastase. Those results show that in hostile environments the stability of L36-hXVNC1ES- is higher than that of the L36-hXVIIINC1ES-.

### III.CONCLUSIONS

1. Recombinant antibody molecules containing the trimerization region from collagen XV NC1 domain are trimeric in solution and recognized the cognate antigen as efficiently as the conventional L36-derived antibody, with the trimerization region from collagen XVIII NC1 domain.
2. Recombinant antibody molecules containing the trimerization region from collagen XV NC1 domain were significantly more stable in serum and in the presence of proteases than conventional L36-derived antibody, with the trimerization region from collagen XVIII NC1 domain.

## Claims

1. A trimeric polypeptide complex comprising three monomer polypeptides, wherein (i) each of said monomer polypeptides comprises a collagen XV trimerizing structural element (XVTSE) and (ii) at least one of said monomer polypeptides is covalently linked to at least one heterologous moiety.

2. Trimeric polypeptide complex according to claim 1, wherein said XVTSE consists of, or comprises, the N-terminal trimerization region of the collagen XV NC1 domain, preferably, a polypeptide having the amino acid sequence shown in SEQ ID NO: 1 [VTAFSNMDDMLQKAHLVIEGTFIYLRDSTEFFIRVRDGWKKLQLGELIPIPADS PPPPALSSNP] or a functionally equivalent variant thereof.

3. Trimeric polypeptide complex according to claim 1, wherein said heterologous moiety is an antibody or a recombinant fragment thereof, or a detectable label.

4. Trimeric polypeptide complex according to claim 1, wherein said monomer polypeptide is linked to said heterologous moiety through a spacer.

5. Trimeric polypeptide complex according to claim 1, wherein said monomer polypeptide is covalently linked to two or more heterologous moieties; wherein said two or more heterologous moieties are, independently, equal or different each other.

6. Trimeric polypeptide complex according to claim 5, wherein
at least one heterologous moiety is positioned N-terminally to said monomer polypeptide and at least one heterologous moiety is positioned C-terminally to said monomer polypeptide, or alternatively, wherein
said two or more heterologous moieties are positioned C-terminally to said monomer polypeptide; or alternatively, wherein
said two or more heterologous moieties are positioned N-terminally to said monomer polypeptide.

7. Trimeric polypeptide complex according to claim 1, which comprises a first monomer polypeptide covalently linked to at least one heterologous moiety and a second monomer polypeptide covalently linked to at least one heterologous moiety.

8. Trimeric polypeptide complex according to claim 1, wherein each one of the three monomer polypeptides is covalently linked to at least one heterologous moiety.

9. A peptide consisting of the sequence SEQ ID NO: 1 [VTAFSNMDDMLQKAHLVIEGTFIYLRDSTEFFIRVRDGWKKLQLGELIPIPADS PPPPALSSNP] or an equivalent variant thereof.

10. A polynucleotide encoding the peptide of claim 9.

11. A polynucleotide encoding a monomer polypeptide, said monomer polypeptide comprising a collagen XV trimerizing structural element (XVTSE) and being covalently linked to at least one heterologous moiety, wherein said heterologous moiety is a polypeptide.

12. A process for producing a trimeric polypeptide complex according to any one of claims 1 to 8, which comprises isolating said trimeric polypeptide complex from a culture comprising a host cell which carries and expresses a polynucleotide according to claim 10 or 11 which encodes at least one of the monomer polypeptides of said trimeric complex, and optionally subjecting the trimeric polypeptide complex to further processing.

13. A vector comprising a polynucleotide according to any one of claims 10 or 11.

14. A pharmaceutical composition comprising a trimeric polypeptide complex according to any one of claims 1 to 8, a polynucleotide according to claim 11, a vector comprising said polynucleotide of claim 11, or a cell comprising said polynucleotide according to claim 11 or said vector comprising said polynucleotide according to claim 11, and at least a pharmaceutically acceptable carrier.

15. Use of a trimeric polypeptide complex according to any one of claims 1 to 8 as a vehicle for assembled antibody fragments to generate a chimeric artificial antibody having preselected pharmacokinetic and/or pharmacodynamic properties.
